# EUROPEAN PATENT APPLICATION

(11) **EP 4 442 217 A1**
(43) Date of publication of application: **09.10.2024**
(21) Application number: 22900700.0
(22) Date of filing: 02.12.2022
(51) Int. Cl.: A61B 18/12, A61B 18/14

(54) **CLOSED-LOOP CONTROL SYSTEM FOR PULSE ABLATION**

(30) Priority: 03.12.2021 CN 202111466101
(71) Applicant: Hangzhouready Biological Technology Co., Ltd, Hangzhou, Zhejiang 311121 (CN)
(72) Inventor: CHEN, Yonggang, Hangzhou, Zhejiang 311121 (CN)
(74) Representative: Cabinet Beau de Loménie
(86) International application number: PCT/CN2022/136315
(87) International publication number: WO 2023/098896

(57) **Abstract**

This application discloses a closed-loop control system for pulse ablation, including an electric pulse host and an electrode group. The electric pulse host includes two paths of control. One path is a control circuit, which is formed by sequentially connecting a main control module with a pulse transmission module, a first electrode control unit and the electrode group. The main control module controls the pulse transmission module to output a pulse signal, and selects a specific electrode through the first electrode control unit to release the pulse signal to act on an ablation site. The other path is a feedback circuit, which is formed by sequentially connecting the main control module with an electrophysiological signal sampling module, a second electrode control unit and the electrode group. The main control module selects an electrode through the second electrode control unit, samples an electrocardiogram signal through the electrophysiological signal sampling module, determines a treatment progress according to the sampled electrocardiogram signal, and adjusts the pulse signal output by the pulse transmission module in real time according to the treatment progress.

## Description

### Technical Field

This application relates to the field of medical devices, and in particular to a closed-loop control system for pulse ablation, which is used for treating arrhythmia and atrial fibrillation.

### Background

The ablation treatment of rapid arrhythmia is achieved through minimally invasive ablation, which damages the myocardial tissues that cause the occurrence of arrhythmia. Minimally invasive ablation is used for treating atrioventricular (nodal) reentrant tachycardia, atrial flutter, atrial fibrillation, tachyarrhythmia, atrial premature beats, and atrial and ventricular premature beats.

Radio-frequency current energy is currently the most commonly used energy source. The electrode at the head of the ablation catheter releases low voltage and high frequency (30kHz-1.5MHz) radio-frequency electric energy, which is converted into thermal energy between the head of the ablation catheter and the local myocardial intima to heat the head of the ablation catheter to 42°C-50°C, thus causing dehydration, degeneration and necrosis of specific local myocardial cells, and change in autonomy and conductivity to cure arrhythmia. Due to thermal conductivity characteristics, it is easy to have adverse effects on the targeted myocardial tissues and other surrounding tissue structures, resulting in esophageal damage including esophageal fistula formation, septal nerve injury, pulmonary vein stenosis, coagulation/thrombus formation, and subsequent thromboembolism causing the risk of cerebral embolism.

Pulse electric field ablation releases a biphasic pulse electric field with high electric field intensity through a direct-current pulse generator, forming a local high voltage difference and releasing non-thermal energy of the high electric field to selectively act on the myocardial area to achieve irreversible electroporation (IRE) of the myocardial cytoplasmic membrane, which causes transmural damage, leakage of cell contents, and death of myocardial cells. The long-term formation of fibrous scars is the same as that of radio-frequency ablation. The myocardial tissues within the strong electric field area are an effective ablation site during pulsed electric field ablation, which can be precisely controlled to reduce the requirement for electrode attachment pressure. It is non-thermal tissue ablation, during which it will not damage the tissue scaffold structure, is not affected by the blood flow "heat sink effect", and will not produce local thrombus ablation. It has a relatively wide therapeutic energy window and tissue resistance specificity. The ablation voltage prioritizes myocardial damage, with minimal and no damage to adjacent tissues such as blood vessels, nerves, and esophagus.

At present, most electric pulse generators work in an open-loop state, can only implement real-time transmission of electric pulses, cannot dynamically optimize and adjust the parameters of electric pulses and electrode discharge according to the ablation effect, thus increasing the risk in treatment.

### Summary

The purpose of this application is to provide a closed-loop control system for pulse ablation, which samples a change amplitude of a potential amplitude signal at an ablation site, adjusts waveform parameters of a pulse signal output by a pulse transmission module according to the corresponding change amplitude to form a control loop, and dynamically optimizes and adjusts the electric pulse parameter and electrode discharge in real time according to the ablation effect. This application discloses a closed-loop control system for pulse ablation, including an electric pulse host and an electrode group including a plurality of electrodes; the electric pulse host including a main control module, a pulse transmission module, an electrophysiological signal sampling module, a first electrode control unit and a second electrode control unit; the main control module being sequentially connected with the pulse transmission module, the first electrode control unit and the electrode group, the main control module controlling the pulse transmission module to output a pulse signal and selecting a specific electrode through the first electrode control unit to release the pulse signal; the main control module being sequentially connected with the electrophysiological signal sampling module, the second electrode control unit and the electrode group, the main control module selecting an electrode through the second electrode control unit and sampling an electrocardiogram signal through the electrophysiological signal sampling module, the electrocardiogram signal referring to a potential amplitude signal between the selected electrodes; the main control module determining a treatment progress according to the sampled electrocardiogram signal and adjusting the pulse signal output by the pulse transmission module in real time according to the treatment progress.

Further, the pulse transmission module and the electrophysiological signal sampling module work alternately and switch once every heartbeat cycle.

Further, the main control module determining a treatment progress according to the sampled electrocardiogram signal and adjusting the pulse signal output by the pulse transmission module in real time according to the treatment progress refers to:
when performing first electrocardiograph signal sampling, if a sampled electrocardiograph signal A is greater than a first threshold, the main control module controlling the pulse transmission module to output a pulse signal according to a set voltage amplitude and pulse width;
for each subsequent electrocardiograph signal sampling, comparing a newly sampled electrocardiograph signal with a previously sampled electrocardiograph signal;
if the newly sampled electrocardiogram signal presents no change, indicating that the position of the corresponding electrode needs to be adjusted;
if the newly sampled electrocardiogram signal presents a change, calculating a change amplitude and correspondingly adjusting waveform parameters of the pulse signal output by the pulse transmission module according to the change amplitude;
when the newly sampled electrocardiogram signal reaches a set second threshold, ending an ablation process.

Further, the closed-loop control system for pulse ablation further includes a voltage and current sensor configured to monitor arc discharge caused by a position change between the electrodes. After the pulse signal is triggered, voltage and current sampling is performed after a certain time delay. Voltage and current sampling is performed in a time shorter than the pulse width. A sampling signal is transmitted in real time to the main control module for analysis after analog-to-digital conversion, and arc determination is performed by determining change characteristics of voltage and current.

Further, the main control module controls the pulse transmission module to release the pulse signal in an electrocardiogram refractory period according to the electrocardiogram signal.

Further, the closed-loop control system for pulse ablation further includes a man-machine interaction module, the man-machine interaction module is connected with the main control module and configured to set parameters, and the parameters include the voltage amplitude and pulse width of the pulse signal.

Further, the pulse transmission module includes a high-voltage power supply and a pulse generation circuit, and the pulse generation circuit achieves a bidirectional pulse by adopting a four-way switch module.

Further, the main control module achieves single-electrode pulse transmission control and single-electrode electrocardiograph signal sampling through the first electrode control unit and the second electrode control unit.

Beneficial effects: (1) according to the data feedback from the electrophysiological signal sampling module, this application determines the treatment progress in the treatment process, thus helping to prevent excessive pulse transmission and incomplete treatment; (2) the cardiac electrophysiological signals can be detected and fed back while performing high-voltage pulse ablation of myocardial cells, the acting pulse waveform parameters including voltage amplitude, pulse width, pulse phase, and the pulse cycle can be adjusted in real time; (3) the arc discharge caused by the position change between the electrodes can be monitored, thus preventing the occurrence of abnormal arcs and making equipment treatment safety higher.

The various technical features disclosed in the summary, the various technical features disclosed in the following embodiments and examples, and the various technical features disclosed in the drawings can be freely combined with each other to form various new technical solutions (these technical solutions should be considered as already recorded in the description), unless the combination of such technical features is technically infeasible.

### Description of the Drawings

FIG. 1 illustrates a schematic diagram of a control system according to this application.
FIG. 2 illustrates a circuit diagram of a pulse transmission module.
FIG. 3 illustrates a schematic diagram of a pulse transmission module and electrophysiological signal sampling module switching circuit.
FIG. 4 illustrates a schematic diagram of a current sampling circuit.
FIG. 5 illustrates a schematic diagram of a voltage sampling circuit.

### Description of the Embodiments

In the following description, many technical details are provided to help readers better understand this application. However, those skilled in the art can understand that even without these technical details and various changes and modifications based on the following embodiments, the technical solution set forth by this application can still be achieved.

In order to make the purpose, technical solution, and advantages of this application clearer, the embodiments of this application will be further described below in detail with reference to the drawings.

Disclosed is a closed-loop control system for pulse ablation, which includes an electric pulse host 100 and an electrode group including a plurality of electrodes 200. One ends of the plurality of electrodes 200 pass through an ablation mapping catheter 300 to reach myocardial tissues (ablation site), while the other ends receive an electric pulse signal and conduct the electric pulse signal to the ablation site.

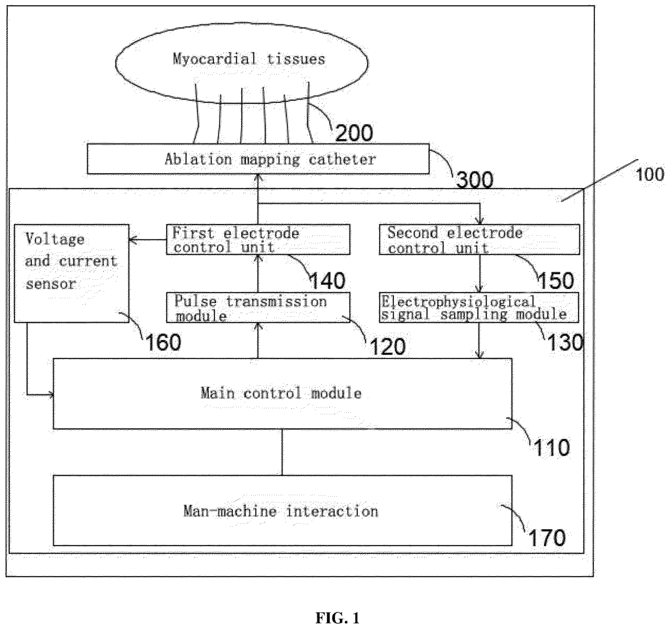

Referring to FIG. 1, the electric pulse host 100 includes a main control module 110, a pulse transmission module 120, an electrophysiological signal sampling module 130, a first electrode control unit 140 and a second electrode control unit 150. The electric pulse host 100 includes two paths of control. One path is a control circuit, which is formed by sequentially connecting the main control module 110 with the pulse transmission module 120, the first electrode control unit 140 and the electrode group. The main control module 110 controls the pulse transmission module 120 to output a pulse signal, and selects a specific electrode through the first electrode control unit 140 to release the pulse signal to act on an ablation site. The other path is a feedback circuit, which is formed by sequentially connecting the main control module 110 with the electrophysiological signal sampling module 130, the second electrode control unit 150 and the electrode group. The main control module 110 selects an electrode through the second electrode control unit 150, samples an electrocardiogram signal through the electrophysiological signal sampling module 130, determines a treatment progress according to the sampled electrocardiogram signal, and adjusts the pulse signal output by the pulse transmission module 120 in real time according to the treatment progress. The main control module can also achieve single-electrode pulse transmission control and single-electrode electrocardiograph signal sampling through the first electrode control unit and the second electrode control unit.

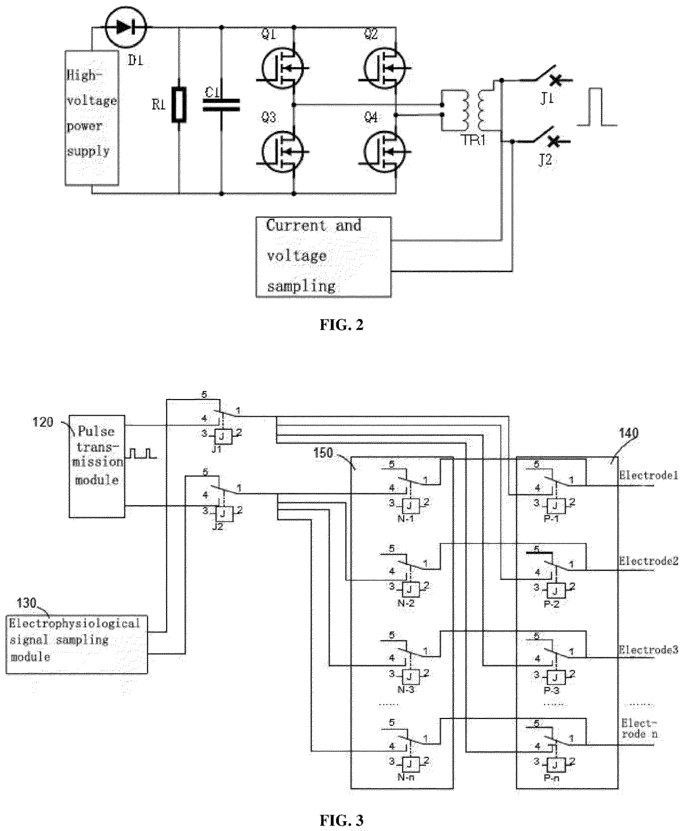

The pulse transmission module includes a high-voltage power supply and a pulse generation circuit. The pulse generation circuit achieves a bidirectional pulse by adopting a four-way switch module. Referring to FIG. 2, when Q1 and Q4 are conducted, the terminal emits a positive-going pulse wave; when Q2 and Q3 are conducted, the terminal emits a negative-going pulse wave. By controlling the conduction time of Q1 and Q4, the positive-going pulse width can be controlled; by controlling the conduction time of Q2 and Q3, the negative-going pulse width can be controlled. The adjustment range of the pulse width is 500ns-50us. By controlling the interval time between two positive-going pulses, the cycle can be controlled, and the pulse cycle is adjustable between 1us and 10ms as needed. By controlling the time interval between positive-going and negative-going pulses, the phase can be controlled. The pulse phase is adjustable between 500ns and 5us.

The pulse transmission module 120 and the electrophysiological signal sampling module 130 work alternately and switch once every heartbeat cycle. By switching the relay corresponding to the electrode, single-electrode pulse transmission control and single-electrode signal sampling can be achieved. The main control module 110 controls the pulse transmission module 120 to release a pulse signal in an electrocardiogram refractory period according to the electrocardiogram signal. Referring to FIG. 3, when pulse transmission is needed, switch contacts of high-voltage vacuum relays J1 and J2 switch from normally closed contacts to normally open contacts, and the electrophysiological signal sampling module 130 is disconnected from the main circuit. When it is required to sample the electrical signal at the electrode, J1 and J2 are at normally closed contacts, and the pulse transmission module 120 is disconnected from the main circuit. A specific electrode can be selected through the backend relay. The electrocardiograph signal sampling module samples the electrocardiograph signal at the ablation site through the electrode to determine the treatment effect. In this embodiment, referring to FIG. 3, the first electrode control unit 140 includes a plurality of relays P_1 to P_n. P_1 to P_n are high-voltage vacuum relays. The number n of the relays is the same as the number of the electrodes. Each relay corresponds to one electrode and is connected with a positive electrode of the electrode. This group of relays is used for positive electrode control of the electric pulse. The second electrode control unit 150 includes a plurality of relays N_1 to N_n. N_1 to N_n are high-voltage vacuum relays. The number n of the relays is the same as the number of the electrodes. Each relay corresponds to one electrode and is connected with a negative electrode of the electrode. This group of relays is used for negative electrode control of the electric pulse. When detecting the electrocardiogram signal, the potential between two electrodes from 1 to n can be arbitrarily selected, or it can be achieved through cyclic scanning. When the detected electrical signal is less than 0.5mV, the pulse transmission stops.

The main control module 110 determines a treatment progress according to the sampled electrocardiogram signal and adjusts the pulse signal output by the pulse transmission module 120 in real time according to the treatment progress. The electrocardiogram signal refers to a potential amplitude signal between the selected electrodes, detected from two adjacent signals, such as electrode 1 and electrode 2, electrode 2 and electrode 3, electrode 3 and electrode 4, till electrode n-1 to electrode n. Specifically, it includes the follows:
(1) when performing first electrocardiograph signal sampling, if a sampled electrocardiograph signal A is greater than a first threshold (5mA), the main control module 110 controls the pulse transmission module 120 to output a pulse signal according to a set voltage amplitude and pulse width (input from a man-machine interaction module 170);
(2) for each subsequent electrocardiograph signal sampling, a newly sampled electrocardiograph signal is compared with a previously sampled electrocardiograph signal; electrocardiogram sampling frequency corresponds to one ablation cycle;
   if the newly sampled electrocardiogram signal presents no change, it indicates that the position of the corresponding electrode needs to be adjusted;
   if the newly sampled electrocardiogram signal presents a change, a change amplitude is calculated and waveform parameters of the pulse signal output by the pulse transmission module 120 are correspondingly adjusted according to the change amplitude; when the newly sampled electrocardiogram signal reaches a set second threshold, an ablation process is ended.

Clinical data show that as the ablation process progresses, the electrocardiogram signal will continue to decrease, that is, the potential amplitude signal measured by the electrodes will gradually decrease. If the newly sampled electrocardiogram signal presents no change, it indicates that the current electrode position may not be the optimal position and needs to be adjusted. When the electrocardiogram signal decreases, it is necessary to compare the decrease amount and decrease amplitude to calculate the percentage of the change amplitude, and the voltage amplitude of the pulse signal or the number of pulses is adjusted according to the change amplitude percentage. When the newly sampled electrocardiogram signal reaches the set second threshold (usually 0.5mV) or less, it indicates that the ablation process is ended.

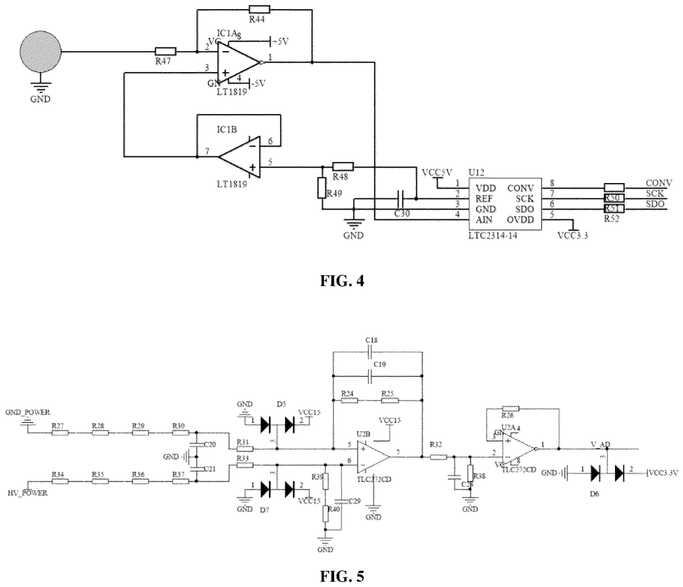

In addition, the control system in this embodiment further includes a voltage and current sensor 160 configured to monitor arc discharge caused by a position change between the electrodes. Arc discharge will cause high ionization pressure and the generation of bubbles, which follow blood flow to the lungs and are very dangerous. Referring to FIG. 2, the voltage and current sensor 160 is located in front of the high-voltage vacuum relays J1 and J2. A separate voltage and current sampling module may be provided, or the voltage and current sampling module may be integrated into the main control module 110. Voltage and current signals are acquired in real time through the voltage and current sensor 160. The acquired signals are transmitted to the main control module 110 and subjected to wavelet transform to monitor whether electrode short-circuits, open-circuits and ignition exist is monitored in real time. If an abnormality is detected, the electrode is cut off in time. A current sampling circuit is as shown in FIG. 4. The current sensor obtains the analog quantity of the current value of the output part, which passes through an operational amplifier modulation circuit and enters an analog-to-digital conversion chip. After the signal is digitized, it is transmitted to the CPLD for processing through timing control. A voltage sampling circuit is as shown in FIG. 5. The sampling differential circuit achieves processing from high voltage to low voltage. After modulation and a certain proportion of attenuation, the voltage signal is directly transmitted to an analog-to-digital conversion interface of the main control module 110.

The arc of the electrode is detected by adopting the following method: after the pulse signal is triggered, voltage and current sampling is performed after a delay of certain time; voltage and current sampling is performed in time smaller than the pulse width, and a sampling signal is transmitted in real time to the main control module 110 for analysis after analog-to-digital conversion. Specifically, when an arc occurs, the voltage decreases, the current will increase, arc determination can be performed by determining change characteristics of the voltage and the current.

In addition, the control system in this embodiment further includes a man-machine interaction module 170 connected with the main control module 110 and configured to set parameters. The parameters include the voltage amplitude and pulse width of the pulse signal.

What are described above are just exemplary embodiment of this application and are not intended to limit the scope of protection of this application. Any modifications, equivalent replacements, improvements and the like made within the spirit and principle of this application shall be included in the scope of protection of this application.

## Claims

1. A closed-loop control system for pulse ablation, comprising an electric pulse host and an electrode group, the electrode group comprising a plurality of electrodes;
the electric pulse host comprising a main control module, a pulse transmission module, an electrophysiological signal sampling module, a first electrode control unit and a second electrode control unit;
the main control module being sequentially connected with the pulse transmission module, the first electrode control unit and the electrode group, the main control module controlling the pulse transmission module to output a pulse signal and selecting a specific electrode through the first electrode control unit to release the pulse signal;
the main control module being sequentially connected with the electrophysiological signal sampling module, the second electrode control unit and the electrode group, the main control module selecting an electrode through the second electrode control unit and sampling an electrocardiogram signal through the electrophysiological signal sampling module, the electrocardiogram signal referring to a potential amplitude signal between the selected electrodes;
the main control module determining a treatment progress according to the sampled electrocardiogram signal and adjusting the pulse signal output by the pulse transmission module in real time according to the treatment progress.

2. The closed-loop control system for pulse ablation according to claim 1, wherein the pulse transmission module and the electrophysiological signal sampling module work alternately and switch once every heartbeat cycle.

3. The closed-loop control system for pulse ablation according to claim 1, wherein the main control module achieves single-electrode pulse transmission control and single-electrode electrocardiograph signal sampling through the first electrode control unit and the second electrode control unit.

4. The closed-loop control system for pulse ablation according to claim 1, 2 or 3, wherein the main control module determining a treatment progress according to the sampled electrocardiogram signal and adjusting the pulse signal output by the pulse transmission module in real time according to the treatment progress refers to: when performing first electrocardiograph signal sampling, if a sampled electrocardiograph signal A is greater than a first threshold, the main control module controlling the pulse transmission module to output a pulse signal according to a set voltage amplitude and pulse width;
for each subsequent electrocardiograph signal sampling, comparing a newly sampled electrocardiograph signal with a previously sampled electrocardiograph signal;
if the newly sampled electrocardiogram signal presents no change, indicating that the position of the corresponding electrode needs to be adjusted;
if the newly sampled electrocardiogram signal presents a change, calculating a change amplitude and correspondingly adjusting waveform parameters of the pulse signal output by the pulse transmission module according to the change amplitude;
when the newly sampled electrocardiogram signal reaches a set second threshold, ending an ablation process.

5. The closed-loop control system for pulse ablation according to claim 1, further comprising a voltage and current sensor configured to monitor arc discharge caused by a position change between the electrodes;
wherein after the pulse signal is triggered, voltage and current sampling is performed after a certain time delay;
voltage and current sampling is performed in a time shorter than the pulse width, a sampling signal is transmitted in real time to the main control module for analysis after analog-to-digital conversion, and arc determination is performed by determining change characteristics of voltage and current.

6. The closed-loop control system for pulse ablation according to claim 1, wherein the main control module controls the pulse transmission module to release the pulse signal in an electrocardiogram refractory period according to the electrocardiogram signal.

7. The closed-loop control system for pulse ablation according to claim 1, further comprising a man-machine interaction module connected with the main control module and configured to set parameters comprising the voltage amplitude and pulse width of the pulse signal.

8. The closed-loop control system for pulse ablation according to claim 1, wherein the pulse transmission module comprises a high-voltage power supply and a pulse generation circuit, and the pulse generation circuit achieves a bidirectional pulse by adopting a four-way switch module.
